# EUROPEAN PATENT APPLICATION

(11) **EP 3 467 098 A1**
(43) Date of publication of application: **10.04.2019**
(21) Application number: 17807096.7
(22) Date of filing: 19.05.2017
(51) Int. Cl.: C12N 1/20, C07K 7/66, A61K 35/74, A61P 31/04, C12R 1/08

(54) **ANEURINIBACILLUS MIGULANUS**

(30) Priority: 01.06.2016 RU 2016121667
(71) Applicant: Joint Stock Company "Valenta Pharmaceuticals", Shchyolkovo, Moskovskaya obl. 141101 (RU)
(72) Inventor: NESTERUK, Vladimir Viktorovich, Moscow 125171 (RU); SYROV, Kirill Konstantinovich, Moscow 121609 (RU)
(74) Representative: Martegani, Franco
(86) International application number: PCT/RU2017/000326
(87) International publication number: WO 2017/209651

(57) **Abstract**

The invention relates to biotechnology and may be used for producing a gramicidin S active substance by using a producing strain of *Aneurinibacillus migulanus,* VKPM B-10212. The obtained mutant strain enables the high-yield production of a gramicidin substance.

## Description

### Field of the Invention

The invention relates to biotechnology and may be used for producing a gramicidin S active substance by using a producing strain *Aneurinibacillus migulanus,* VKPM B-10212. The obtained mutant strain enables the high-yield production of the gramicidin substance.

### Background of the Invention

Known are methods for the production of gramicidin S comprising submerged culturing a *Bacillus brevis* producing strain on synthetic media [V.V. Korshunov Physiology of metabolism of Bacillus brevis subsp. G.-B. in association with biosynthesis of gramicidin S.- Candidate's dissertation, 1962, Moscow, MSU, V.V. Korshunov, N.S. Egorov Synthetic medium for Bacillus brevis culturing and grammidin producing. Mikrobiologiia, 1962; 31: 3, 515-519, V.N. Shaposhnikov, I.L. Rabotnova, A.B. Silaev et. al. Method for producing crystalline gramicidin S. - Inventor's certificate No. 187243, 1963, M.B. Kupletskaya Effect of aeration on grammidin development and production by culture of Bacillus brevis var. G.-B. Mikrobiologiia, 1965; 34: 5, 905-909, G.G. Zharikova Natural variability of spore bacteria and biosynthesis of polypeptide antibiotics. - Doct. diss., 1972, Moscow, MSU, A.I. Berezovskaya, A.N. Vypiyach, N.S. Egorov et. al. Bacillus brevis strain 101 - gramicidin S-producing. - Inventor's certificate No. 686463, 1978, Method for submerged culturing *Bacillus brevis* strain 101 for producing gramicidin S, -RU2447143, 11/24/2008].

Known is a periodic process for producing gramicidin S comprising culturing the most productive *Bacillus brevis* strains [T.P. Udalova Features of Bacillus brevis var. G.-B growth and development associated with biosynthesis of gramicidin S. - Cand. Diss., 1979, Moscow, MSU], which was used for the synthesis of the product when culturing *Bacillus brevis* strain 101 [T.P. Yudina Physiological and biochemical features of grammidin-producing Bacillus brevis subsp. G.-B. and variants thereof. - Cand. Diss., 2002, Moscow, MSU], is characterized by performing a single load of a nutrient medium into a reactor, seeding with an inoculum, culturing while aerating and stirring for 27 hours until a pH of 6.0 and less is reached.

The object of the invention is to create a more efficient method for producing gramicidin with an industrially high yield and to provide microorganisms that can be used in the method. The object is achieved by providing a novel strain of *Aneurinibacillus migulanus,* VKPM B-10212.

### Description of the Invention

In one embodiment, the invention relates to the *Aneurinibacillus migulanus* bacterium strain VKPM B-10212.

In a preferred embodiment, the invention relates to the *Aneurinibacillus migulanus* bacterium strain VKPM B-10212, gramicidin-producing.

In another embodiment, the invention relates to use of the *Aneurinibacillus migulanus* bacterium strain VKPM B-10212 for the gramicidin production.

The generic and specific name of the culture: *Aneurinibacillus migulanus.*

The access number: VKPM B-10212.

The method: the strain was created as a mutant.

The culture was identified by RNCIM FSUI "GosNIIGenetika" (Moscow), the identification report on the strain No. 101 using 16S RNA analysis method is dated on March 16, 2009. The deposit certificate is attached. Date of deposit: 03/03/2009.

Cultural and morphological features of the strain: rods of 4 to 8 µm long, 0.6 to 0.7 µm wide, the strain produces spores with oval ribs of 0.8 to 1.0 µm. It is aerobic. It does not fluidify gelatin. It does not hydrolyze starch. It uses nitrogen in an amine and ammonium form. When cultured on the Gauze and Brazhnikova's agar growth medium for 40-48 hours at a temperature of 40±1 °C, it forms round-shaped colonies, prominent above the agar surface, with a well-defined crateriform or pointlike center. Colonies are beige-colored, opaque and have a pasty consistency. They can be easily removed from the agar surface using an inoculation loop. The shape and size of the colonies vary greatly depending on the medium composition, the seeding density and other factors. During the culturing process, it can segregate into variants having a distinct morphology and reduced antibiotic production.

Application field for the strain: industrial production of antibiotics.

The product synthesized by the strain: gramicidin S hydrochloride (crystalline).

When cultured in shake flasks at a liquid "Routine Laboratory" medium for 72 hours, the strain activity (productivity), other production performance, produces 2±0.3 g/L gramicidin S corresponding to 0.2±0.02 g_{gramicidin S}/g_{biomass}. When grown in fermenters under optimal conditions, the specific production of gramicidin S can be up to 0.4 g_{gramicidin S}/g_{biomass}. The average productivity of the strains maintained in the active state on agar media as of 01/27/2009 was 2,073 g/L gramicidin S; related impurity content was 15.3%.

A method for the strain activity determination including the technique: gramicidin S extraction from the culture liquid with ethyl alcohol, followed by determination of gramicidin S concentration by a high-performance liquid chromatography technique.

Method, conditions and medium composition for the strain long-term storage: storing the spores on millet, storing the vegetative cells and spores in a freeze-dried state (with 10 to 20% milk).

Method, conditions and media composition for the strain extension: growing on the Gauze and Brazhnikova's agar medium (yeast water of 50 vol. % or 28 mg% based on amine nitrogen, 1% peptone, 0.5% NaCl, 3% agar-agar, pH 7.0) for 48-72 hours at 40 °C. Culturing on the liquid semi-synthetic "Routine Laboratory" medium (per 1 liter of the medium: 20 mL distilled glycerol, 8 mL food grade lactic acid 40%, 9.9 g K₂HPO₄, 5 g NaCl, 0.2 g MgSO₄·7H₂O, 8.12 g ammonium oxalate, up to 5 mg% yeast autolysate) in 0.75 L shake flasks (having 0.1 L medium) on an orbital shaker (rotation speed of 220 rpm, 1 m amplitude) at 40 °C for 48-72 hours.

Optimal conditions and the fermentation medium composition: "Fermentation Routine" medium (30 mL glycerol, 24.6 mL lactic acid 40%, 2 g K₂HPO₄, 5 g NaCl; 0.2 g MgSO₄·7H₂O, 12 g ammonia oxalate, up to 10 mg% yeast autolysate, up to 20 mg% casein hydrolyzate, 2 mL Laprol, pH after sterilization between 7.02 and 7.2). The aeration is to be 1.25 m³ O₂/m³ of the medium per hour; the pressure is to be 0.5 ATMG; the stirring intensity should provide the oxygen mass transfer at 0.2-0.3 mmol O₂/L•min at the start of fermentation to 0.4-1.0 mmol O₂/L•min at the end of the fermentation, depending on the biomass achieved (0.035 to 0.045 mmol O₂/g_{biomass}•min). The chemostating is to be at pH of 6.4 to 7.2. The culturing is to be for 16 to 48 hours, depending on the fermentation method. DNA of the strain comprises a HindIII restriction site.

The strain is not zoopathogenic, phytopathogenic.

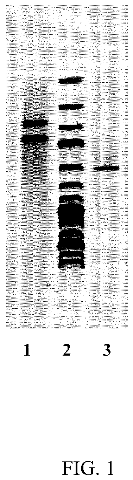

Fig. 1. Restriction analysis of the DNA fragment encoding 16S RNA gene in the test strain 101.
1. The DNA fragment encoding 16S RNA gene of the test strain 101, treated with HindIII restrictase.
2. Marker 1 kb DNA Ladder (10000, 8000, 6000, 5000, 4000, 3500, 3000, 2500, 2000, 1500, 1000, 750, 500, 250 bp, bottom-upwards)
3. The DNA fragment encoding 16S RNA gene of the test strain 101, not treated with HindIII restrictase.

### Example 1. Strain 101 Identification to Species using 16S RNA Assay

### Work Stages

**I. Screening the culture until a single colony and obtaining biomass for 16S RNA assay**
**II. Isolating the DNA**
**III. Selecting the PCR primers and regimens**
   Conservative primers for 16S rDNA production:
   **8f** - aga gtt tga tcc tgg ctc ag
   **926r** - ccg tca att cct ttr agt tt
   **1492r** - ggt tac cct tgt tac gac tt
   with reaction regimens:
   1. 95 °C for 3 min.
   2. 35 cycles
      95 °C for 30 sec.
      57 °C for 30 sec.
      72 °C for 1 min. 30 sec.
   3. 72 °C for 5 min.
**IV. 16S rDNA Sequencing, Sequence Comparison and Kinship Tree Construction**
   Sequencing is performed on the automatic sequencer AE3000.
   For the sequence analysis, specialized phylogenetic computer programs are used.
   **Stability of Result Reproduction.** PCR reactions are performed in at least three replicates.
   **Conditions for Sample PCR Electrophoresis.**
   1.0% agarose gel, electrophoresis at an electric field of 5 V/cm.
**V. Restriction analysis of the DNA fragment encoding 16S RNA gene**

### a) Sequencing of 16S rDNA variable regions.

Sequencing of 16S rDNA variable regions provided the following assembled nucleotide sequence for the strain **101:**

atgaaggttttcgatcgtaaagttctgttgttagggaagaaccgccggggatgacctccc
cggtctgacggttncctaaacgagaaagcccccggctaatctacggtgcccagcagccgc
ggtaatacgtagggggcaagcgttgtccggaattattgggcgtaaagcgcgcgcaggcgg
cttcttaagtcaggtgtgaaagcccacggctcaaccgtggagggccacttgaaactggga
agcttgagtgcaggagaggagagcggaattccacgtgtagcggtgaaatgcgtagagatg
tggaggaacacccgtggcgaaggcggctctctggcctgtaactgacgctgaggcgcgaaa
gcgtggggagcgaacaggattagataccccctggtagtccacgccgtaaacgttgagtgct
aggtgttggggactccaatcctcagtgctcgcagctaacgcaat

aagcactccgcctggg

### b) Analysis of Sequencing Results and Kinship Tree Construction

Primary screening against GenBank and RDP-II databases showed that the test strain belongs to the following systematic groups: Bacteria; Firmicutes; Bacillales; Paenibacillaceae; Aneurinibacillus group; Aneurinibacillus, the homology with the species *Aneurinibacillus migulanus* and *Aneurinibacillus aneurinilyticus* being 98%.

Sequences were aligned with the corresponding sequences from the most related species of bacteria available from the GenBank database.

The sequence results processed with the computer program located on the website of RDB II (Ribosomal Database Project II), which serves to determine the kinship between microorganisms and constructing the phylogenetic trees, are represented graphically.

**Table 1. Distance matrix**

| | | 1 | 2 | 3 | 4 | 5 | 6 | 7 | 8 | 9 | 10 |
|---|---|---|---|---|---|---|---|---|---|---|---|
| **101.txt** | 1 | --- | 0.980 | 0.987 | 0.958 | 0.991 | 0.892 | 0.907 | 0.989 | 0.958 | 0.989 |
| **Ab.anurly3** | 2 | 0.020 | --- | 0.985 | 0.955 | 0.989 | 0.895 | 0.909 | 0.991 | 0.955 | 0.991 |
| **Ab.migula** | 3 | 0.013 | 0.015 | --- | 0.962 | 0.996 | 0.902 | 0.912 | 0.994 | 0.962 | 0.994 |
| **Ab.thaerph** | 4 | 0.042 | 0.045 | 0.038 | --- | 0.967 | 0.903 | 0.912 | 0.965 | 1 | 0.965 |
| **Ab.migula2** | 5 | 0.009 | 0.011 | 0.004 | 0.033 | --- | 0.902 | 0.917 | 0.998 | 0.967 | 0.998 |
| **B.badius2** | 6 | 0.108 | 0.105 | 0.098 | 0.097 | 0.098 | --- | 0.889 | 0.905 | 0.903 | 0.905 |
| **Oxl.oxali2** | 7 | 0.093 | 0.091 | 0.088 | 0.088 | 0.083 | 0.111 | --- | 0.919 | 0.912 | 0.919 |
| **Ab.anurly2** | 8 | 0.011 | 0.009 | 0.006 | 0.035 | 0.002 | 0.095 | 0.081 | --- | 0.965 | 1 |
| **Ab.thaerp2** | 9 | 0.042 | 0.045 | 0.038 | 0.000 | 0.033 | 0.097 | 0.088 | 0.035 | --- | 0.965 |
| **Ab.anurlyt** | 10 | 0.011 | 0.009 | 0.006 | 0.035 | 0.002 | 0.095 | 0.081 | 0.000 | 0.035 | --- |

**Table 2. Sequence Description**

| | |
|---|---|
| Ab.migula2 | *Aneurinibacillus migulanus* DSM 2895 (T) |
| Ab.anurly2 | *Aneurinibacillus aneurinilyticus* DSM 5562 (T) |
| Ab.migulan | *Aneurinibacillus migulanus* ATCC 9999 (T) |
| Ab.anurlyt | *Aneurinibacillus aneurinilyticus* NCIMB 10056 |
| Ab.anurly3 | *Aneurinibacillus aneurinilyticus* ATCC 12856 (T) |
| Ab.thaerph | *Aneurinibacillus thermoaerophilus* str. L420-91 DSM 10154 (T) |
| Ab.thaerp2 | *Aneurinibacillus thermoaerophilus* DSM 10155 |
| Oxl.oxali2 | *Oxalophagus oxalicus* str. Alt Ox1 DSM 5503 (T) |
| B.badius2 | *Bacillus badius* ATCC 14574 (T) |
| 101.txt | Test strain 101 |

Further analysis against 16S rRNA RDP II database showed homology with the same species of bacteria, and the most related species are *Aneurinibacillus migulanus* and *Aneurinibacillus aneurinilyticus.*

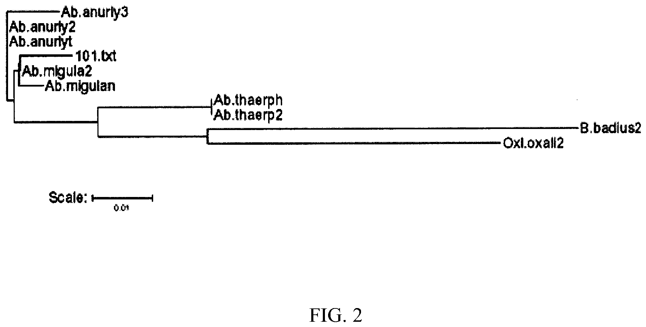

Based on the analysis data, a phylogenetic tree with homologous strains was constructed (Fig. 2).

The relevancy criterion a microorganism to a particular type is at least 97% homology. Using this criterion, the test strain can be referred to *Aneurinibacillus migulanus* or *Aneurinibacillus aneurinilyticus* species.

For more accurate identification, the restriction analysis of the DNA fragment encoding 16S RNA gene was used.

This DNA fragment in *Aneurinibacillus migulanus* is reported to comprise HindIII restriction site, whereas *Aneurinibacillus aneurinilyticus* comprise no this restriction site.

Restriction analysis was performed for the DNA fragment encoding 16S RNA gene in the test strain 101. The analysis results are shown in Fig. 1.

The presence of 620 bp and 870 bp fragments after the DNA fragment treatment with HindIII restrictase indicates that HindIII site is comprised in the DNA fragment from the test strain 101. This criterion allows to refer the test strain 101 to *Aneurinibacillus migulanus* species.

### Example 2. Method for producing gramicidin S

Gramicidin S is produced using the fermentation technique. The method consists of four stages: treating the productive strain of *Aneurinibacillus migulanus,* VKPM B-10212, in a laboratory, preparing a vaccine material, culturing an inoculum in a laboratory fermenter, providing the biosynthesis of gramicidin C in a main fermenter.

The main parameters of fermentation were selected based on the morphology and stability of the productive strain of *Aneurinibacillus migulanus,* VKPM B-10212. Time for productive strain culturing on an agar medium in an incubator ranges from 48 to 120 hours and allows the inoculum in a larger volume compared to culturing on Petri dishes. The bacterial suspension is stored in a 15% protective glycerol solution at a temperature of between -20 °C and -80 °C.

Preliminary preparation of the inoculum is performed in a laboratory fermenter on an agar medium and in the absence of other microorganisms.

The inoculum is then transferred to the next fermenter in an amount of 1 to 2.5% by volume of the fermentation medium consisting of sodium citrate, ammonium chloride, potassium dihydrogen phosphate, D- and L-lactic acid in a 1:1 ratio, yeast extract powder, casein powder, defoaming agent Struktol J 647. Optimal parameters for the fermentation process were optical density from 10 to 20, pH from 5.5 to 7.5, the absence of other microorganisms. The fermentation process takes from 48 to 120 hours.

The next production stage involves fermenting the inoculum in the main fermenter. The inoculum volume is 8 to 17% of the fermentation medium consisting of sodium citrate, ammonium chloride, potassium dihydrogen phosphate, D- and L-lactic acid in a 1:1 ratio, yeast extract powder, casein powder, defoaming agent Struktol J 647. The optimal pH value in the range of 6.5 to 7.0 is controlled by adding the ammonia solution. The optimal nutrient concentration is maintained by adding the glycerol, ammonium sulfate and cornstarch solutions. The fermentation process takes from 48 to 120 hours.

The produced biomass is filtered with a ceramic filter unit using an ultrafiltration method, washing the biomass with deionized water to remove impurities from the biomass. The concentrated biomass is dried in a spray dryer at 180 °C. The dried biomass is washed with acetone at 40 °C to remove colored impurities. Acetone is removed by purging with nitrogen followed by one more rinsing with acetone and drying under vacuum at 45 °C

Further, gramicidin C is extracted with ethanol, adjusting the pH to 3.0-4.0 with diluted hydrochloric acid (HCl:ethanol = 1:2). Extraction is performed repeatedly, removing the extracts with pressurized nitrogen. After ethanol separation, the biomass is dried under vacuum at about 47 °C.

The dried biomass is dissolved in water and decolorized with activated carbon followed by filtering on a filter press, rinsing with ethanol to reduce product loss through the sorption on the carbon.

The obtained extract is transferred into a crystallizer, diluted with deionized water, added with concentrated sodium chloride solution and heated to the temperature of 55 to 65 °C. After clarification, the solution is cooled to a temperature of not above 5 °C. The obtained crystals are separated in a centrifuge and dried under vacuum at a temperature of at most 65 °C, further pin milled, sieved through 400 and 200 µm mesh screens and packed in polyethylene bags placed in aluminum containers.

## Claims

1. *Aneurinibacillus migulanus* bacterium strain VKPM B-10212, gramicidin-producing.

2. Use of *Aneurinibacillus migulanus* bacterium strain VKPM B-10212 for the gramicidin production.


Sequence listing

<110>  JOINT STOCK COMPANY "VALENTA PHARMACEUTICALS"

<120>  ANEURINIBACILLUS MIGULANUS STRAIN AND USE THEREOF
<130>  PCT/RU2017/000326
<160>  1

<210>  1
<211>  480
<212>  rDNA
<213>  Aneurinibacillus migulanus, strain VKPMB-10212

<400>  1
atgaaggttt tcgatcgtaa agttctgttg ttagggaaga accgccgggg atgacctccc   60
cggtctgacg gttncctaaa cgagaaagcc cccggctaat ctacggtgcc cagcagccgc   120
ggtaatacgt agggggcaag cgttgtccgg aattattggg cgtaaagcgc gcgcaggcgg   180
cttcttaagt caggtgtgaa agcccacggc tcaaccgtgg agggccactt gaaactggga   240
agcttgagtg caggagagga gagcggaatt ccacgtgtag cggtgaaatg cgtagagatg   300
tggaggaaca cccgtggcga aggcggctct ctggcctgta actgacgctg aggcgcgaaa   360
gcgtggggag cgaacaggat tagatacccc tggtagtcca cgccgtaaac gttgagtgct   420
aggtgttggg gactccaatc ctcagtgctc gcagctaacg caataagcac tccgcctggg   480

Sequence listing

<110>  JOINT STOCK COMPANY "VALENTA PHARMACEUTICALS"

<120>  ANEURINIBACILLUS MIGULANUS STRAIN AND USE THEREOF
<130>  EP17807096.7, based on PCT/RU2017/000326
<160>  4

<210>  1
<211>  20
<212>  DNA primer
<213>  Artificial sequence
<220>  Conservative 8f primer for 16S rDNA production

<400>  1
agagtttgat cctggctcag   20

<210>  2
<211>  20
<212>  DNA primer
<213>  Artificial sequence
<220>  Conservative 926r primer for 16S rDNA production

<400>  2
ccgtcaattc ctttragttt   20

<210>  3
<211>  20
<212>  DNA primer
<213>  Artificial sequence
<220>  Conservative 1492r primer for 16S rDNA production

<400>  3
ggttaccctt gttacgactt   20

<210>  4
<211>  480
<212>  rDNA
<213>  Aneurinibacillus migulanus, strain VKPMB-10212

<400>  4
atgaaggttt tcgatcgtaa agttctgttg ttagggaaga accgccgggg atgacctccc   60
cggtctgacg gttncctaaa cgagaaagcc cccggctaat ctacggtgcc cagcagccgc   120
ggtaatacgt agggggcaag cgttgtccgg aattattggg cgtaaagcgc gcgcaggcgg   180
cttcttaagt caggtgtgaa agcccacggc tcaaccgtgg agggccactt gaaactggga   240
agcttgagtg caggagagga gagcggaatt ccacgtgtag cggtgaaatg cgtagagatg   300
tggaggaaca cccgtggcga aggcggctct ctggcctgta actgacgctg aggcgcgaaa   360
gcgtggggag cgaacaggat tagatacccc tggtagtcca cgccgtaaac gttgagtgct   420
aggtgttggg gactccaatc ctcagtgctc gcagctaacg caataagcac tccgcctggg   480